# FASCICULE DE BREVET EUROPEEN

(11) **EP 1 347 736 B1**
(45) Date de publication et mention de la délivrance du brevet: **07.02.2007**
(21) Numéro de dépôt: 01994913.0
(22) Date de dépôt: 19.12.2001
(51) Int. Cl.: A61Q 5/04, A61Q 5/08, A61Q 5/10, A61K 8/81

(54) **COMPOSITION OXYDANTE POUR LE TRAITEMENT DES MATIERES KERATINIQUES A BASE DE POLYMERES AMPHIPHILES D'AU MOINS UN MONOMERE A INSATURATION ETHYLENIQUE A GROUPEMENT SULFONIQUE ET COMPORTANT UNE PARTIE HYDROPHOBE**
OXIDATIVE ZUSAMMENSETZUNG ZUR BEHANDLUNG KERATINISCHER MATERIALIEN AUF DER BASIS VON AMPHIPHILEN POLYMEREN, DIE MINDESTENS EIN ETHYLENISCH UNGESÄTTIGTES SULFONSÄUREGRUPPEN TRAGENDES MONOMER UND EINEN HYDRPHOBEN TEIL ENTHALTEN
OXIDISING COMPOSITION FOR TREATING KERATINOUS MATERIALS BASED ON AMPHIPHILIC POLYMERS OF AT LEAST AN ETHYLENICALLY SULFONATED UNSATURATED MONOMER WITH SULPHONIC GROUP AND COMPRISING A HYDROPHOBIC PART

(30) Priorité: 22.12.2000 FR 0016954; 11.01.2001 FR 0100328
(43) Date de publication de la demande: 01.10.2003
(73) Titulaire: L'ORÉAL, 75008 Paris (FR)
(72) Inventeur: KRAVTCHENKO, Sylvain, F-92600 Asnieres (FR); LAGRANGE, Alain, F-77700 Coupvray (FR)
(74) Mandataire: Wattremez, Catherine
(86) Numéro de dépôt international: PCT/FR2001/004077
(87) Numéro de publication internationale: WO 2002/051369

(56) Documents cités:
- EP-A- 0 750 899
- FR-A- 2 753 372
- US-A- 6 110 451

## Description

La présente invention a trait à une composition oxydante gélifiée, destinée au traitement des matières kératiniques comprenant un polymère amphiphile selon revendication 1 ainsi que ses utilisations pour la teinture, pour la déformation permanente ou pour la décoloration des fibres kératiniques humaines et en particulier des cheveux.

Il est connu de décolorer les fibres kératiniques et en particulier les cheveux humains, avec des compositions de décoloration contenant un ou plusieurs agents oxydants. Parmi les agents oxydants classiquement utilisés, on peut citer le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.
Lesdites compositions de décoloration se présentent principalement sous forme de produits anhydres (poudres ou crèmes) contenant des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.
Les compositions de décoloration peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.
Les compositions de décoloration se présentent également sous forme de compositions aqueuses épaissies de peroxyde d'hydrogène, prêtes à l'emploi.

Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

Il est connu par ailleurs de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants. On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.
L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène ou des composés susceptibles de produire le peroxyde d'hydrogène par hydrolyse tels que le peroxyde d'urée, les persels comme les perborates et persulfates. Le peroxyde d'hydrogène est particulièrement préféré.

Pour localiser le produit de décoloration ou de teinture à l'application sur les cheveux afin qu'il ne coule pas sur le visage ou en dehors des zones que l'on se propose de décolorer, on a jusqu'ici eu recours à l'emploi d'épaississants traditionnels tels que l'acide polyacrylique réticulé, les hydroxyéthylcelluloses, certains polyuréthanes, les cires, et en outre, dans le cas des compositions aqueuses de décoloration, à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.
Cependant, la demanderesse a constaté que les systèmes épaississants mentionnés ci-dessus ne permettaient pas d'obtenir des décolorations suffisamment puissantes et homogènes et laissaient les cheveux rêches.
Par ailleurs, elle a également constaté que les compositions de décoloration prêtes à l'emploi contenant le ou les agents oxydants, et en outre les systèmes épaississants de l'art antérieur ne permettaient pas une application suffisamment précise sans coulures ni chutes de viscosité dans le temps.

On sait également que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.
Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique ou l'acide thioglycolique, leurs sels ainsi que leurs esters, notamment le thioglycolate de glycérol.
Concernant les compositions oxydantes nécessaires à la mise en oeuvre de l'étape de fixation, on fait le plus souvent appel, dans la pratique, à des compositions à base d'eau oxygénée.

Par ailleurs, on recherche depuis de nombreuses années des formulations cosmétiques sous forme de gels transparents. Ce type de présentation est très appréciée par le consommateur pour des raisons esthétiques et pour des raisons de facilité et de confort d'utilisation.
La forme gel correspond le plus souvent à une préoccupation pratique pour le formulateur : faciliter la prise du produit hors de son conditionnement sans perte significative, limiter la diffusion du produit à la zone locale de traitement et pouvoir l'utiliser dans des quantités suffisantes pour obtenir l'effet cosmétique recherché. Cet objectif est important pour les formulations oxydantes utilisées en teinture capillaire, pour les permanentes ou pour la décoloration des cheveux. Celles-ci doivent bien s'étaler et se répartir de façon régulière le long des fibres kératiniques et ne pas couler sur le front, la nuque, le visage ou dans les yeux.
Il est généralement difficile d'obtenir des gels à base de peroxyde tel que le peroxyde d'hydrogène stables à la conservation en utilisant des épaississants et/ou des gélifiants hydrosolubles classiques, par exemple ceux du type polymère acrylique réticulé comme ceux vendus sous le nom CARBOPOL® par la société GOODRICH. Les peroxydes sont utilisés en cosmétique sous forme de solutions aqueuses acides pour des raisons de stabilité. Ils conduisent le plus souvent en présence des gélifiants classiques à des variations sensibles de la viscosité du gel durant le stockage.

On connaît dans le brevet US 4.804.705. des gels à base de peroxyde d'hydrogène contenant un gélifiant formé par réaction d'une hydroxyéthylcellulose quaternisée du type CELQUAT® (produit vendu par NATIONAL STARCH), d'une solution aqueuse à 15% en poids d'un polymère poly(acide 2-acrylamido 2-méthylpropane sulfonique) non-réticulé du type COSMEDIA HSP-1180® (produit vendu par HENKEL) et d'un polystyrène sulfonate de sodium du type FLEXAN 3® (produit vendu par NATIONAL STARCH), utilisés dans des concentrations particulières.

FR 2 753 372 décrit une composition cosmétique destinée au traitement des matières kératiniques contenant un polymère amphiphile comme un poly(acide-2-acrylamido-2-mithylpropan sulfonique) réticulé par exemple par le triméthylol propane triacrylate et un oxydant.

La demanderesse a découvert de manière surprenante qu'il est possible d'obtenir des compositions de décoloration prêtes à l'emploi qui ne coulent pas et restent donc bien localisées au point d'application, et qui permettent aussi d'obtenir des décolorations puissantes et homogènes tout en laissant les cheveux moins rêches si on introduit dans la composition une quantité efficace d'un polymère amphiphile particulier.

La demanderesse a également découvert de manière surprenante une nouvelle famille d'agents épaississants et/ou gélifiants permettant d'obtenir des gels transparents à base d'oxydants et de préférence de peroxyde d'hydrogène ou de composé oxydant susceptible de libérer du peroxyde d'hydrogène, stables au stockage. Il s'agit de polymère amphiphile comportant au moins un monomère à insaturation éthylénique à groupement sulfonique, sous forme libre ou partiellement ou totalement neutralisée et en outre au moins une partie hydrophobe. Ces nouveaux agents gélifiants n'affectent pas les propriétés oxydantes du peroxyde d'hydrogène ou d'un composé susceptible de produire le peroxyde d'hydrogène par hydrolyse présent dans le gel ainsi formé.

La présente invention a donc pour objet une composition cosmétique et/ou dermatologique destinée au traitement des matières kératiniques comprenant dans un support approprié pour les matières kératiniques :
(a) au moins un polymère amphiphile. Selon la revendication 1, comportant au moins un monomère à insaturation éthylénique à groupement sulfonique, sous forme libre ou partiellement ou totalement neutralisée et en outre au moins une partie hydrophobe et,
(b) au moins un oxydant.

L'invention concerne également l'utilisation de ces polymères comme agent épaississant et/ou gélifiant dans des compositions cosmétiques et/ou dermatologiques comprenant au moins un oxydant.

Selon l'invention l'agent oxydant est de préférence choisi dans le groupe constitué par le peroxyde d'hydrogène et les composés susceptibles de produire par hydrolyse du peroxyde d'hydrogène, ou leurs mélanges.

Mais d'autres caractéristiques, aspects, objets et avantages de l'invention apparaîtront encore plus clairement à la lecture de la description et des exemples qui suivent.

### Polymères amphiphiles selon l'invention

Les polymères conformes à l'invention sont des polymères amphiphiles comportant au moins un monomère à insaturation éthylènique à groupement sulfonique, sous forme libre ou partiellement ou totalement neutralisée et comprenant au moins une partie hydrophobe tels que définis à la revendication 1.

On entend par polymère amphiphile, tout polymère comportant à la fois une partie hydrophile et une partie hydrophobe et notamment une chaîne grasse.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non-réticulés.
De préférence, on choisit des polymères amphiphiles réticulés.
Lorsqu'ils sont réticulés, les agents de réticulation peuvent être choisis parmi les composés à polyinsaturation oléfinique couramment utilisés pour la réticulation des polymères obtenus par polymérisation radicalaire.
On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétraallyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Dans les polymères conformes à l'invention, le monomère à insaturation éthylénique à groupement sulfonique est l'acide 2-acrylamido-2-méthylpropane-sulfonique (AMPS) ainsi que ses formes partiellement ou totalement neutralisées.

Les polymères amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères de l'invention sont choisis parmi les copolymères amphiphiles d'AMPS et d'au moins un monomère hydrophobe à insaturation éthylénique comportant au moins une partie hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.
Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 2.20-221».

Les monomères hydrophobes à insaturation éthylénique de ces copolymères particuliers sont choisis parmi les acrylates ou les acrylamides de formule (I) suivante : 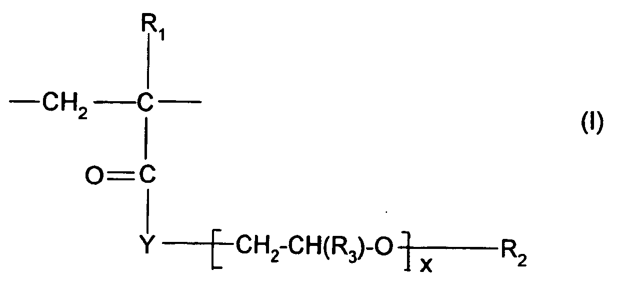 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)); les radicaux alkylperfluorés en C₆-C₁₈ (par exemple le groupement de formule -(CH₂)₂-(CF₂)₉-CF₃); le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (I) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578.

On peut également citer les copolymères non-réticulés et réticulés d'AMPS partiellement ou totalement neutralisé et de méthacrylate de dodécyle ainsi que les copolymères non-réticulés et réticulés d'AMPS partiellement ou totalement neutralisé et de n-dodécylméthacrylamide, tels que ceux décrits dans les articles de Morishima cités ci-dessus.

On citera plus particulièrement les copolymères constitués de motifs acide 2-acrylamido-2-méthylpropane-sulfonique (AMPS) de formule (II) suivante : 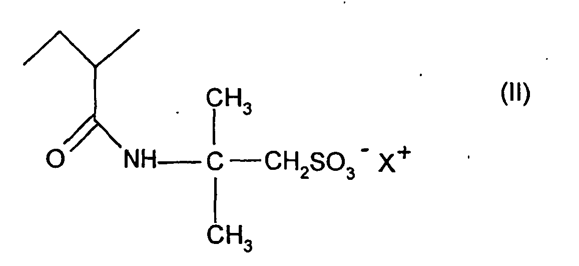 dans laquelle X⁺ est un proton, un cation de métal alcalin, un cation alcalino-terreux ou l'ion ammonium,
et de motifs de formule (III) suivante : 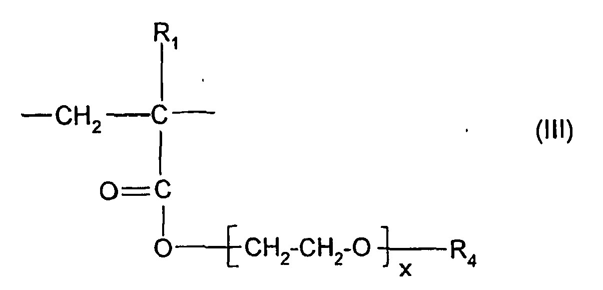 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

Les polymères particulièrement préférés sont ceux pour lesquels x = 25 , R₁ désigne méthyle et R₄ représente n-dodécyle ; ils sont décrits dans les articles de Morishima mentionnés ci-dessus.
Les polymères pour lesquels X⁺ désigne sodium ou ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.
En utilisant la polymérisation par précipitation dans le tert-butanol, il est possible d'obtenir une distribution de la taille des particules du polymère particulièrement favorable pour ses utilisations.

La distribution de la taille des particules du polymère peut être déterminée par exemple par diffraction laser ou analyse d'image.
Une distribution intéressante pour de type de polymère et déterminée par analyse d'image est la suivante: 60,2% inférieur à 423 microns, 52,0% inférieur à 212 microns, 26,6% inférieur à 106 microns, 2,6% inférieur à 45 microns et 26,6% supérieur à 850 microns.
La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.
Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL^{®} C-080 de la société HOECHST/CLARIANT),
- d'un alcool oxo en C₁₁ oxyéthyléné par 8 moles d'oxyde d'éthylène (GENAPOL^{®} UD-080 de la société HOECHST/CLARIANT),
- d'un alcool oxo en C₁₁ oxyéthyléné par 7 moles d'oxyde d'éthylène (GENAPOL^{®} UD-070 de la société HOECHST/CLARIANT),
- d'un alcool en C₁₂-C₁₄ oxyéthyléné par 7 moles d'oxyde d'éthylène (GENAPOL^{®} LA-070 de la société HOECHST/CLARIANT),
- d'un alcool en C₁₂-C₁₄ oxyéthyléné par 9 moles d'oxyde d'éthylène (GENAPOL^{®} LA-090 de la société HOECHST/CLARIANT),
- d'un alcool en C₁₂-C₁₄ oxyéthyléné par 11 moles d'oxyde d'éthylène (GENAPOL^{®} LA-110 de la société HOECHST/CLARIANT),
- d'un alcool en C₁₆-C₁₈ oxyéthyléné par 8 moles d'oxyde d'éthylène (GENAPOL^{®} T-080 de la société HOECHST/CLARIANT),
- d'un alcool en C₁₆-C₁₈ oxyéthyléné par 15 moles d'oxyde d'éthylène (GENAPOL^{®} T-150 de la société HOECHST/CLARIANT),
- d'un alcool en C₁₆-C₁₈ oxyéthyléné par 11 moles d'oxyde d'éthylène (GENAPOL^{®} T-110 de la société HOECHST/CLARIANT),
- d'un alcool en C₁₆-C₁₈ oxyéthyléné par 20 moles d'oxyde d'éthylène (GENAPOL^{®} T-200 de la société HOECHST/CLARIANT),
- d'un alcool en C₁₆-C₁₈ oxyéthyléné par 25 moles d'oxyde d'éthylène (GENAPOL^{®} T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% en moles.
De préférence pour les polymères les plus hydrophobes, la proportion molaire en motifs de formule (I) ou (III) varie de 50,1 à 99,9%, plus particulièrement de 70 à 95% et encore plus particulièrement de 80 à 90%.
De préférence pour les polymères peu hydrophobes, la proportion molaire en motifs de formule (I) ou (III) varie de 0,1 à 50%, plus particulièrement de 5 à 25% et encore plus particulièrement de 10 à 20%.
La distribution des monomères dans les polymères de l'invention peut être, par exemple, alternée, bloc (y compris multibloc) ou quelconque.
Selon l'invention, on préfère que les polymères aient des chaînes pendantes sensibles à la chaleur et dont la solution aqueuse présente une viscosité qui, au delà d'une certaine température seuil, augmente, ou demeure pratiquement constante quand la température augmente.
Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.

Ces polymères, de préférence, conduisent dans l'eau à un phénomène de démixion par chauffage se traduisant par des courbes présentant, en fonction de la température et de la concentration, un minimum appelé LCST (Lower Critical Solution Temperature).

Les viscosités (mesurées à 25°C au viscosimètre Brookfield aiguille 7) des solutions aqueuses à 1% vont de préférence de 20 000 mPas à 100 000 mPas et plus particulièrement de 60 000 mPas à 70 000 mPas.

Les polymères amphiphiles conformes à l'invention sont présents dans les compositions dans des concentrations allant de 0,01 à 30% en poids, plus préférentiellement de 0,1 à 10%, encore plus préférentiellement de 0,1 à 5% en poids et plus particulièrement encore de 0,5 à 2% en poids.

### Agent oxydant

L'agent oxydant de la composition selon l'invention est choisi de préférence dans le groupe formé par l'eau oxygénée, le peroxyde d'urée, les persels tels que les perborates ou les persulfates ou leurs mélanges.

De préférence, l'agent oxydant est le peroxyde d'hydrogène, et plus préférentiellement encore, l'agent oxydant est de l'eau oxygénée.

La concentration en peroxyde d'hydrogène peut varier de 0,5 à 40 volumes, de préférence de 2 à 30 volumes et celle en composé susceptible de former du peroxyde d'hydrogène par hydrolyse de 0,1 à 25 % en poids par rapport au poids total de la composition oxydante.

Les compositions oxydantes selon l'invention peuvent être anhydres ou aqueuses.

Les compositions oxydantes selon l'invention sont de préférence aqueuses et le pH de l'ensemble d'une composition oxydante aqueuse varie de préférence de 1 à 13, et encore plus préférentiellement de 2 à 12.

La composition oxydante peut également se présenter, en particulier dans le cas de la décoloration, sous forme de deux parties à mélanger au moment de l'emploi, l'une de ces deux parties contenant des agents alcalins et se présentant sous forme solide ou liquide. Pour le peroxyde d'hydrogène, le pH est de préférence inférieur à 7 avant mélange.

Le pH des compositions aqueuses oxydantes selon l'invention peut être obtenu et/ou ajusté classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

La composition oxydante peut contenir des additifs connus pour leur utilisation dans les compositions oxydantes pour la teinture des cheveux par oxydation, pour la déformation permanente ou la décoloration des cheveux tels que des agents alcalinisants ou acidifiants, des agents conservateurs, des agents séquestrants tel que l'EDTA et l'acide étidronique, des filtres UV, des cires, des silicones volatiles ou non, cycliques ou linéaires ou ramifiées, organomodifiées (notamment par des groupements amines) ou non, des céramides, des pseudocéramides, des huiles végétales, minérales ou de synthèse, les vitamines ou provitamines comme le panthénol, des opacifiants, etc....

De préférence, lorsque l'agent oxydant est de l'eau oxygénée, la composition oxydante selon l'invention contient au moins un agent stabilisant de l'eau oxygénée. Dans les compositions associant l'eau oxygénée et les polymères amphiphiles de la présente invention, des résultats particulièrement avantageux ont été obtenus en utilisant au moins un agent stabilisant choisi parmi les pyrophosphates des métaux alcalins ou alcalino-terreux, les stannates des métaux alcalins ou alcalino-terreux, la phénacétine ou les sels d'acides et d'oxyquinoléine, comme le sulfate d'oxyquinoléine. De manière plus avantageuse encore, on utilise au moins un stannate en association ou non à au moins un pyrophosphate.
On peut également utiliser l'acide salicylique et ses sels, l'acide pyridine dicarboxylique et ses sels, le paracétamol et les systèmes constitués a) d'un tampon [borate de métal alcalin (Na, K) ou d'ammonium et de préférence tétraborate de Na, décahydraté], b) d'un agent alcalin (NH₄OH, monoéthanolamine, carbonate d'ammonium, carbonate acide d'ammonium, soude), et c) d'un agent séquestrant d'ion métallique lourd (Fe, Mn, Cu) tels que ceux décrits dans les demandes de brevets WO-01/72271, WO-01/72272, WO-01/52801.

Dans les compositions oxydantes selon l'invention, la concentration en agents stabilisants de l'eau oxygénée peut varier de 0,0001 % à 5% en poids et de préférence de 0,01 à 2% en poids par rapport au poids total des compositions oxydantes.
Dans les compositions oxydantes selon l'invention à l'eau oxygénée, le rapport des concentrations du peroxyde d'hydrogène aux agents stabilisants peut varier de 0,05 à 1000 et de préférence de 0,1 à 500 et plus préférentiellement encore de 1 à 200. De même, le rapport des concentrations du ou des polymère(s) amphiphile(s) selon l'invention aux agents stabilisants peut varier de 0,05 à 1000 et de préférence de 0,1 à 500 et plus préférentiellement encore de 1 à 200.
Dans les compositions oxydantes selon l'invention la concentration en agents oxydants varie de 0,1 à 25% en poids par rapport au poids total de la composition.
De préférence, selon l'invention, le rapport des concentrations du ou des polymère(s) amphiphile(s) selon l'invention aux agents oxydants est compris entre 0,001 et 10, les quantités desdits polymères et oxydants étant exprimées en matières actives (peroxyde d'hydrogène pour l'eau oxygénée). Plus préférentiellement encore, ce rapport est compris entre 0,01 et 5 et plus particulièrement encore entre 0,02 et 1.

Lorsque les compositions selon l'invention se présentent généralement sous la forme d'un gel transparent, leur viscosité varie de préférence de 50 mPa.s à 10 Pa.s et plus préférentiellement de 75 mPa.s à 0,5 Pa.s.

Plus particulièrement, les compositions selon l'invention peuvent comprendre en outre au moins un polymère substantif amphotère ou cationique.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 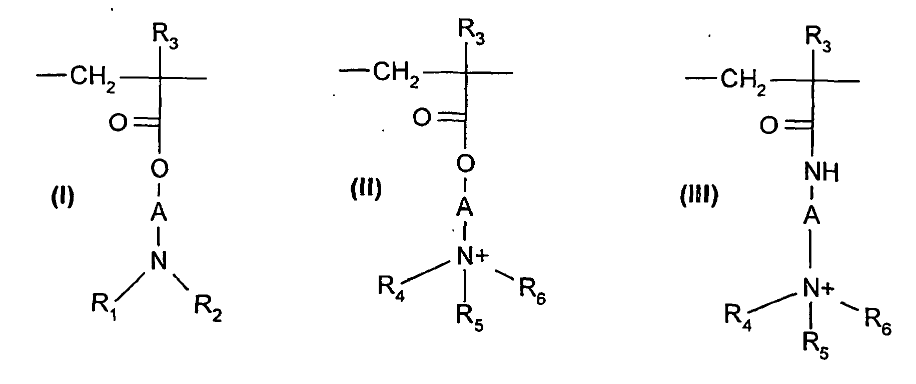 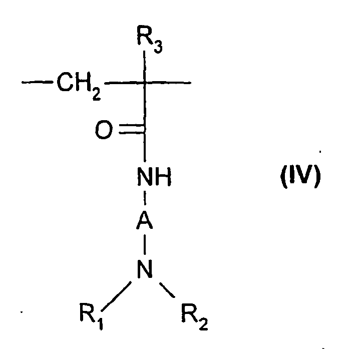 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP.
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les *hydroxyméthyl-, hydroxyéthyl- ou hydroxypropyl* celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 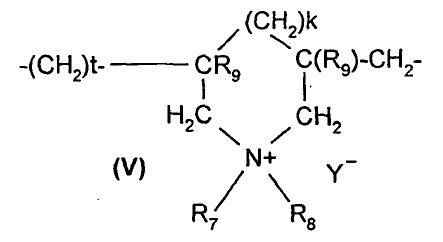 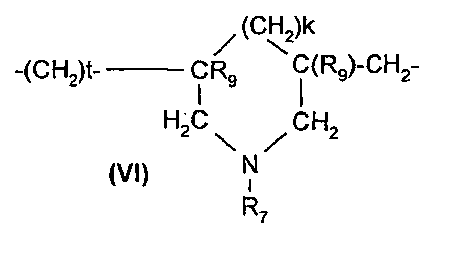 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 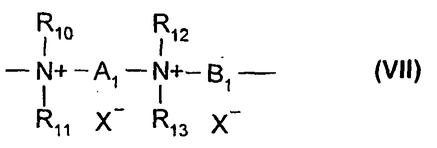 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :
         -(CH₂-CH₂-O)x-CH₂-CH₂-
         -[CH₂-CH(CH₃)-O]y-CH₂-CH(CH₃)-
         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂- ;
      d) un groupement uréylène de formule : -NH-CO-NH-.

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 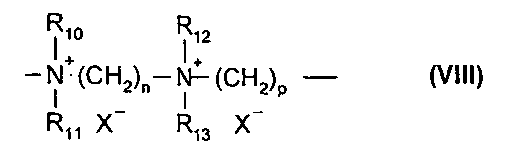 dans laquelle R₁₀, R₁₁, R₁₂ et R₁₃, identiques ou différents, désignent un radical alkyle
   ou hydroxyalkyle ayant de 1 à 4 atomes de carbone environ, n et p sont des nombres entiers variant de 2 à 20 environ et, X- est un anion dérivé d'un acide minéral ou organique.
(11) Les polyammoniums quaternaires constitués de motifs récurrents de formule (IX) : 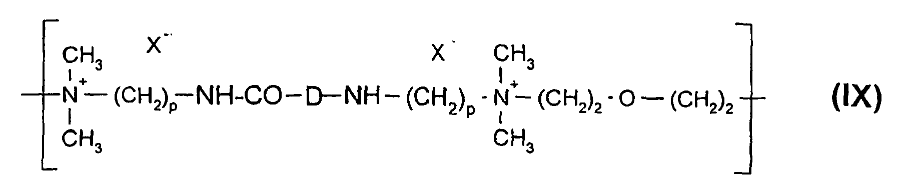 dans laquelle p désigne un nombre entier variant de 1 à 6 environ, D peut être nul ou peut représenter un groupement -(CH₂)ᵣ -CO- dans lequel r désigne un nombre égal à 4 ou à 7, X- est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de "POLYETHYLENEGLYCOL (15) TALLOW POLYAMINE " dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de "SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 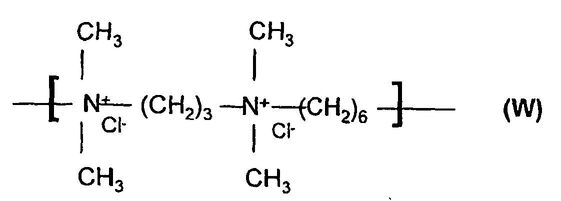 et notamment ceux dont le poids moléculaire, déterminé par chromatographie par perméation de gel, est compris entre 9500 et 9900; 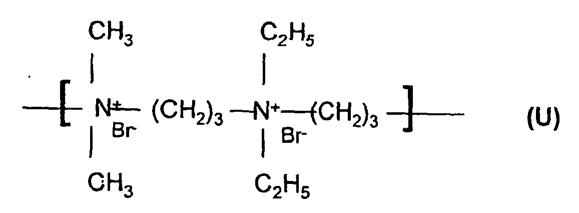 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes
ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale : 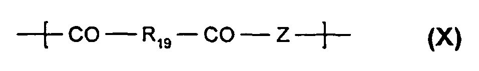 dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 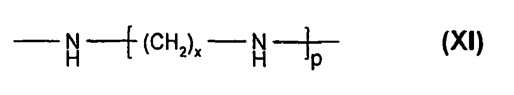 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 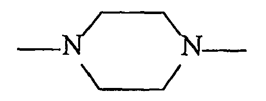
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

   Les alcanes sultones utilisées dans l'alcoylation sont de préférence la propane ou la butane sultone, les sels des agents d'alcoylation sont de préférence les sels de sodium
   ou de potassium.
(4) Les polymères comportant des motifs zwittérioniques de formule : 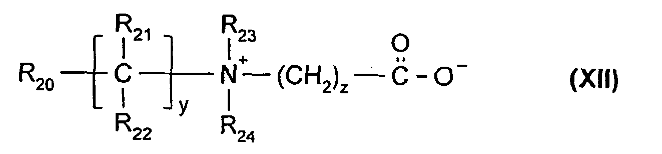 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 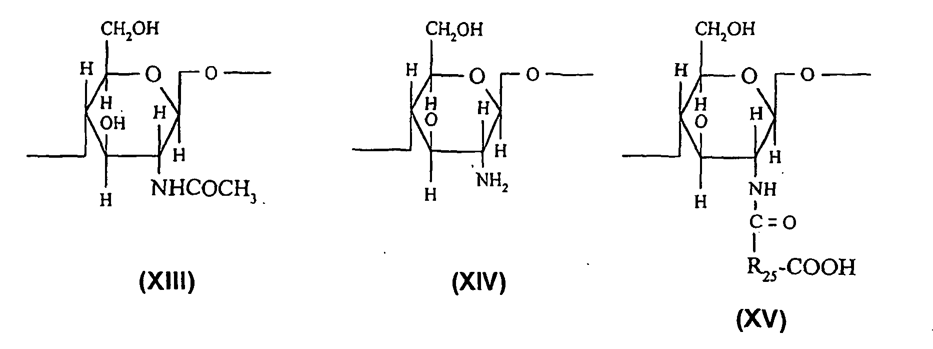 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 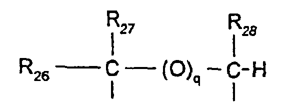 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine
   ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
   Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical -CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 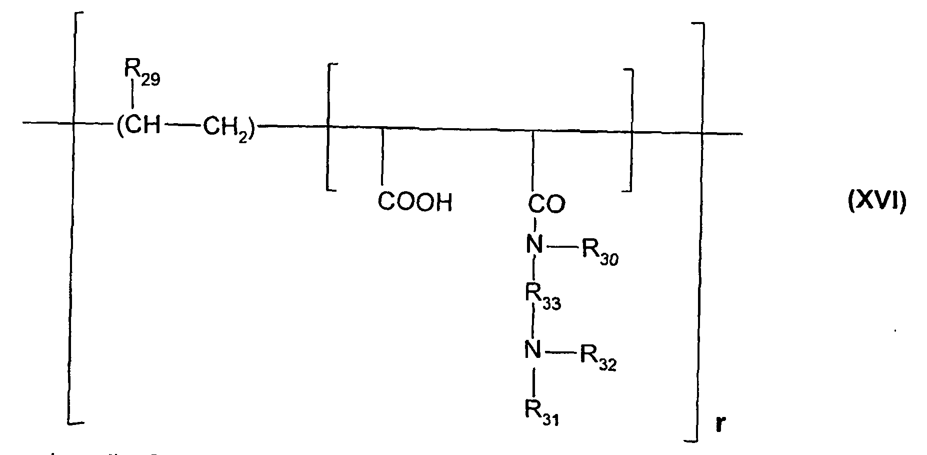 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 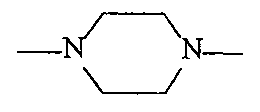 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)
   où D désigne un radical 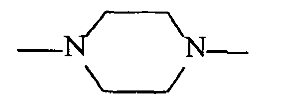 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs.
Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside. sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂-CHOH-SO₃H
R₂' désigne un radical alkyle d'un acide Rg -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Les compositions selon l'invention peuvent également comprendre d'autres agents d'ajustement de la rhéologie tels que les épaississants cellulosiques (hydroxyéthycellulose, hydroxypropylcellulose, carboxyméthylcellulose..), la gomme de guar et ses dérivés(hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique et les polymères associatifs non ioniques, anioniques, cationiques ou amphotères, tels que les polymères commercialisés sous les appellations PEMULEN TR1 ou TR2 par la société GOODRICH, SALCARE SC90 par la société ALLIED COLLOIDS, ACULYN 22, 28, 33, 44, ou 46 par la société ROHM & HAAS et ELFACOS T210 et T212 par la société AKZO.

Ces épaississants d'appoint peuvent représenter de 0,01 à 10% en poids du poids total de la composition.

Un autre objet de l'invention est un procédé de teinture d'oxydation des fibres kératiniques humaines et en particulier des cheveux mettant en oeuvre une composition colorante comprenant dans un support approprié pour la teinture desdites fibres au moins un colorant d'oxydation et une composition oxydante telle que définie ci-dessus.

Selon ce procédé, on applique sur les fibres au moins une composition colorante telle que définie précédemment, la couleur étant révélée à pH acide, neutre ou alcalin à l'aide d'une composition oxydante selon l'invention qui est appliquée simultanément ou séquentiellement, avec ou sans rinçage intermédiaire.

Selon une forme de mise en oeuvre particulièrement préférée du procédé de teinture selon l'invention, on mélange, au moment de l'emploi, la composition colorante décrite ci-dessus avec une composition oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Un autre objet de la présente invention est un procédé de déformation permanente des fibres kératiniques humaines et en particulier des cheveux, utilisant comme composition oxydante la composition définie ci-dessus.
Selon ce procédé, on applique sur la fibre kératinique à traiter une composition réductrice, la fibre kératinique étant mise sous tension mécanique avant, pendant ou après ladite application, on rince éventuellement la fibre, on applique sur la fibre éventuellement rincée la composition oxydante de la présente invention puis on rince éventuellement à nouveau la fibre.
La première étape de ce procédé consiste à appliquer sur les cheveux une composition réductrice. Cette application se fait mèche par mèche ou globalement.
La composition réductrice comprend au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.
L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.
Les cheveux peuvent également être mis en forme sans l'aide de moyens extérieurs, simplement avec les doigts.
Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape, facultative, du procédé (étape (ii)), les cheveux imprégnés de la composition réductrice sont donc ensuite rincés soigneusement par une composition aqueuse.

Puis, dans une troisième étape (étape (iii)), on applique sur les cheveux ainsi rincés la composition oxydante selon la présente invention, dans le but de fixer la nouvelle forme imposée aux cheveux.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées selon l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.

On obtient finalement une chevelure facile à démêler, douce. Les cheveux sont ondulés.

La composition oxydante selon l'invention peut également être utilisée dans un procédé de décoloration des fibres kératiniques humaines et en particulier des cheveux.

Le procédé de décoloration selon l'invention comprend une étape d'application sur les fibres kératiniques d'une composition oxydante selon l'invention, cette composition comprenant de préférence de l'eau oxygénée en milieu alcalin après mélange extemporané. Classiquement, une deuxième étape du procédé de décoloration selon l'invention est une étape de rinçage des fibres kératiniques.

Les exemples suivants illustrent l'invention sans présenter un caractère limitatif.

### EXEMPLES DE PREPARATION:

### Préparation des esters (méth)acryliques éthoxylés.

Ils peuvent être notamment obtenus par action de (méth)acrylate de glycidyle, ou d'acide (méth)acrylique, ou d'un (méth)acrylate d'alkyle, ou d'un halogénure de (méth)acryloyle sur un alcool gras éthoxylé. On peut citer, à titre d'exemples non limitatifs, les préparations suivantes :
a) à partir du méthacrylate de glycidyle et du GENAPOL T-250
b) à partir de l'acide (méth)acrylique et du GENAPOL UD-070
c) à partir du (méth)acrylate de méthyle et du GENAPOL LA-090
d) à partir du chlorure de (méth)acryloyle et du GENAPOL UD-070.

a) Dans un réacteur tricol d'un litre équipé d'un agitateur, d'un thermomètre et d'un condensateur à reflux, on introduit 500g de Genapol T-250 et 75g de méthacrylate de glycidyle. On chauffe le mélange réactionnel à la température de 100°C pendant 2 heures, et on élimine l'excès de méthacrylate de glycidyle par distillation sous pression réduite. Le monomère obtenu peut être utilisé pour la polymérisation sans purification ultérieure.
b) Dans un réacteur tricol d'un litre équipé d'un agitateur, d'un thermomètre et d'un condensateur à reflux, on introduit 500g de Genapol UD-070, 100g d'acide (méth)acrylique et de l'acide p-toluènesulfonique comme catalyseur. On chauffe le mélange réactionnel au reflux pendant 2 heures, et l'on sépare l'excès d'acide et d'eau formée au cours de la réaction par distillation sous pression réduite. Le monomère obtenu peut être utilisé pour la polymérisation sans purification ultérieure.
c) Dans un réacteur tricol d'un litre équipé d'un agitateur, d'un thermomètre et d'un condensateur à reflux, on introduit 500g de Genapol LA-090, 100g de (méth)acrylate de méthyle et 20g de tétraisopropoxyde de titane. On chauffe le mélange réactionnel au reflux pendant 2 heures, et après séparation par distillation de l'alcool formé, on distille l'ester qui reste sous pression réduite.
   Le monomère obtenu peut être utilisé pour la polymérisation sans purification ultérieure.
d) Dans un réacteur tricol d'un litre équipé d'un agitateur, d'un thermomètre et d'un condensateur à reflux, on introduit 500g de Genapol UD-070, 110g de chlorure de (méth)acryloyle et 50g de carbonate de sodium. On chauffe le mélange réactionnel au reflux pendant 2 heures, et l'on sépare l'excès de chlorure d'acide par distillation sous pression réduite. Le monomère obtenu peut être utilisé pour la polymérisation sans purification ultérieure.

### Polymérisation selon la méthode de précipitation dans le tert-butanol

Dans un réacteur de 2 litres muni d'un condensateur à reflux, d'une prise de gaz, d'un thermomètre et d'un agitateur, on introduit 500ml de tert-butanol, et la quantité calculée d'AMPS. On neutralise le mélange en introduisant du NH3, et on ajoute le monomère préparé ci-dessus au mélange réactionnel. On rend le mélange réactionnel inerte par passage d'azote ou d'argon et lorsque la température intérieure a atteint 60°C, on introduit l'initiateur (AIBN) pour amorcer la polymérisation.
Après quelques minutes, le polymère ainsi préparé précipite. On porte le mélange à reflux pendant 2 heures, et on sépare le polymère du solvant par filtration à la trompe, puis on le sèche sous pression réduite.

De la façon décrite ci-dessus, on a préparé les polymères suivants :
(à partir des réactants suivants en des quantités exprimées en grammes)

| | | | | |
|---|---|---|---|---|
| Méthacrylate de Genapol T-250 ..... | 10 | 20 | 30 | 97 |
| AMPS neutralisé par NH3..... | 90 | 80 | 90 | 3 |
| Méthylène-bis-acrylamide (réticulant).. | | | 1,5 | |
| Méthacrylate d'allyle (réticulant)......... | | 1,7 | | |
| TMPTA (réticulant)..... | 1,8 | | | 1,8 |
| Azobisisobutyronitrile (initiateur)...... | | | 1 | |
| Peroxyde dilauryle (initiateur)......... | 1 | 1 | | 1 |
| Tert-butanol..... | 300 | 300 | 300 | 300 |

### EXEMPLE 1: Composition de teinture d'oxydation:

### Composition colorante :

On a utilisé la nuance châtain foncé de la gamme du commerce
EXCELLENCE CREME® de L'OREAL.

### Composition oxydante:

- Copolymère acide acrylamido-2-méthyl-2-propane-sulfonique/n-dodécylacrylamide (3.5%/96,5%) neutralisé à 100% par la soude..... 1 g
- Peroxyde d'hydrogène ............q.s....................................... 20 volumes
- Pyrophosphate tétrasodique (0,02g) et stannate de sodium (0,04 g)
- Agent séquestrant : pentaacétate pentasodique.....0,06 MA*
- Eau déminéralisée..... q.s.p..... 100g
MA* désigne Matière Active

Au moment de l'emploi, la composition colorante a été mélangée à la composition oxydante dans un rapport pondéral 1/1,5.
Le mélange obtenu a été appliqué sur des mèches de cheveux gris à 90 % de blancs à raison de 10 g pour 1 g de cheveux , et laissé poser 30 minutes.
Les cheveux ont ensuite été rincés, lavés avec un shampooing standard puis séchés.

On a obtenu une nuance châtain foncé uniforme.

### EXEMPLE 2: Composition de déformation permanente:

On a préparé la composition réductrice suivante (exprimée en grammes) :

| | |
|---|---|
| Acide thioglycolique | 9,2 |
| Arginine | 15 |
| Ammoniaque à 20% d'NH3 | 9,3 |
| Carbonate d'ammonium | 4,5 |
| Cocoylamidopropylbétaïne/monolaurate de glycérol (25/5) en solution aqueuse à 30% | 1,3 |
| Peptisant | 0,8 |
| Alcool isostéarylique (TEGO ALKANOL 66 vendu par la société GOLDSCHMIDT)..... | 12 |
| Agent séquestrant | 0,4 |
| Parfu | 0,4 |
| Eau déminéralisée q.s.p. | 100 |

Cette composition réductrice a été appliquée sur une mèche de cheveux humides, préalablement enroulée sur un bigoudi de 9 mm de diamètre.
Après 10 minutes de temps de pause, on l'a rincée abondamment à l'eau.
On a ensuite appliqué la composition oxydante suivante :

### Composition oxydante:

- Copolymère acide acrylamido-2-méthyl-2-propane-sulfoniquel n-dodécylacrylamide (3,5%196,5%) neutralisé à 100% par la soude 1 g
- Eau oxygénée 8 volumes
- Pyrophosphate tétrasodique (0,02g) et stannate de sodium (0,04 g)
- Agent séquestrant: pentaacétate pentasodique 0,06 MA*
- Eau déminéralisée q.s.p. 100g
MA* désigne Matière Active

Après 10 minutes de temps de pause, on a rincé à nouveau abondamment la mèche.
Puis on a déroulé les cheveux du bigoudi et on les a séchés.
La mèche a été ondulée.

Des résultats similaires ont été obtenus en remplaçant le copolymère acide acrylamido-2-méthyl-2-propane-suifonique/n-dodécylacrylamide neutralisé par la soude des exemples 1 et 2 ci-dessus par un copolymère réticulé par du méthylène-bis-acrylamide constitué de 75% en poids de motifs AMPS neutralisés par NH₃ et de 25% en poids de motifs de formule (III) dans laquelle R₁=H, R₄=C₁₆-C₁₈ et x=25.

Des résultats similaires ont été obtenus en remplaçant le copolymère acide acrylamido-2-méthyl-2-propane-sulfonique/n-dodécylacrylamide neutralisé par la soude des exemples 1 et 2 ci-dessus par un copolymère réticulé par du méthacrylate d'allyle constitué de 90% en poids de motifs AMPS neutralisés par NH₃ et de 10% en poids de motifs méthacrylate de GENAPOL T-250 [motifs de formule (III) dans laquelle R₁=CH₃, R₄=C₁₆-C₁₈ et x=25], ou par un copolymère réticulé par du méthacrylate d'allyle constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25].

## Revendications

1. Composition cosmétique et/ou dermatologique destinée au traitement des matières kératiniques, **caractérisée par le fait qu'**elle comprend dans un support approprié pour les matières kératiniques :
(a) au moins un polymère amphiphile comportant au moins un monomère à insaturation éthylénique à groupement sulfonique, sous forme libre ou partiellement ou totalement neutralisée et en outre au moins une partie hydrophobe choisi parmi les copolymères amphiphiles d'AMPS et d'au moins un monomère hydrophobe à insaturation éthylénique comportant au moins une partie hydrophobe ayant de 6 à 50 atomes de carbone choisi parmi les acrylates ou les acrylamides de formule (I) suivante : 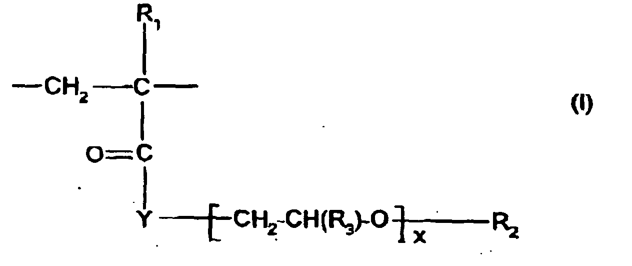 dans laquelle R₁ et R₃, identiques ou différents, désignent un atome d'hydrogène ou un radical alkyle linéaire ou ramifié en C₁-C₆ ; Y désigne O ou NH ; R₂ désigne un radical hydrocarboné hydrophobe comportant au moins de 6 à 50 atomes de carbone; x désigne un nombre de moles d'oxyde d'alkylène et varie de 0 à 100 et,
(b) au moins un oxydant.

2. Composition selon la revendication 1, **caractérisée par le fait que** R₂ désigne un radical hydrocarboné hydrophobe comportant de 6 à 22 atomes de carbone.

3. Composition selon la revendication 2, **caractérisée par le fait que** R₂ désigne un radical hydocarboné hydrophobe comportant au 6 à 18 atomes de carbone.

4. Composition selon la revendication 3, **caractérisée par le fait que** R₂ désigne un radical hydrocarboné hydrophobe comportant de 12 à 18 atomes de carbone.

5. Composition selon l'une quelconque des revendications 1 à 4, **caractérisée par le fait que** les polymères amphiphiles sont neutralisés partiellement ou totalement par une base minérale ou organique.

6. Composition selon l'une quelconque des revendications 1 à 5, **caractérisée par le fait que** les polymères amphiphiles ont un poids moléculaire moyen en nombre allant de 1000 à 20 000 000 g/mole.

7. Composition selon la revendication 6, **caractérisée par le fait que** le poids moléculaire moyen en nombre varie de 20 000 à 5 000 000 g/mole.

8. Composition selon la revendication 7, **caractérisée par le fait que** le poids moléculaire moyen en nombre varie de 100 000 à 1 500 000 g/mole.

9. Composition selon l'une quelconque des revendications précédentes, **caractérisée par le fait qu'**une solution aqueuse à 1% en poids desdits polymères présente à la température de 25°C une viscosité mesurée au viscosimètre Brookfield, aiguille 7, allant de 20 000 mPas à 100 000 mPas

10. Composition selon l'une quelconque des revendications précédentes, **caractérisée par le fait que** les polymères amphiphiles sont préparés par polymérisation radicalaire par précipitation dans le tert-butanol.

11. Composition selon l'une quelconque des revendications précédentes, **caractérisée par le fait que** les polymères amphiphiles sont réticulés ou non-réticutés.

12. Composition selon la revendication 11, **caractérisée par le fait que** les polymères amphiphiles sont réticulés.

13. Composition selon la revendication 12, **caractérisée par le fait que** le ou les agents de réticulation sont choisis parmi les composés à polyinsaturation oléfinique.

14. Composition selon la revendication 13, **caractérisée par le fait que** le ou les agents de réticulation sont choisis parmi le méthylène-bis-acrylamide, le méthacrylate d'allyle ou le triméthylol propane triacrylate (TMPTA).

15. Composition selon rune quelconque des revendications 12 à 14, **caractérisée par le fait que** le taux de réticulation varie de préférence de 0.01 à 10% en moles.

16. Composition selon l'une quelconque de revendications 1 à 15, **caractérisée par le fait que** les polymères amphiphiles sont choisis parmi les polymères statistiques d'AMPS modifiés par réaction avec une n-mono(C₈-C₂₂)alkylamine ou une di-n-(C₈-C₂₂)alkylamine.

17. Composition selon l'une quelconque des revendications 1 à 16, **caractérisée par le fait que** les polymères amphiphiles d'AMPS contiennent en plus au moins un monomère à insaturation éthylénique ne comportant pas de chaîne grasse.

18. Composition selon la revendication 17, **caractérisée par le fait que** le monomère à insaturation éthylénique ne comportant pas de chaîne grasse est choisi parmi les acides (méth)acryliques et leurs dérivés alkyl substitués en β, et leurs esters obtenus avec des monoalcools ou des mono- ou polyalkylèneglycols, ou bien parmi les (méth)acrylamides, la vinylpyrrolidone, l'anhydride maléique, l'acide itaconique ou l'acide maléique, ou les mélanges de ces composés.

19. Composition selon l'une quelconques de revendications 1 à 18, **caractérisée par le fait que** le radical hydrophobe R₂ est choisi parmi les radicaux alkyles en C₈-C₁₈, linéaires, ramifiés ou cycliques ; les radicaux alkylperfluorés en C₈-C₁₈ ; le radical cholestéryle ou un ester de cholestérol; les groupes polycycliques aromatiques.

20. Composition selon l'une quelconque des revendications 1 à 19, **caractérisée par le fait que** le monomère de formule (I) comporte en plus au moins un motif oxyde d'alkylène (x≥1).

21. Composition selon l'une quelconque des revendications 1 à 20, **caractérisée par le fait que** le monomère de formule (I) comporte en plus au moins une chaîne polyoxyalkylénée.

22. Composition selon la revendication 21, **caractérisée par le fait que** la chaîne polyoxyalkylénée est constituée de motifs oxyde d'éthylène et/ou de motifs oxyde de propylène.

23. Composition selon la revendication 22, **caractérisée par le fait que** la chaîne polyoxyalkylénée est constituée uniquement de motifs oxyde d'éthylène.

24. Composition selon l'une quelconque des revendications 1 à 23, **caractérisée par** la fait que le nombre de motifs oxyalkylénés varie de 3 à 100.

25. Composition selon la revendication 24, **caractérisée par le fait que** le nombre de motifs oxyalkylénés varie de 3 à 50.

26. Composition selon la revendication 25, **caractérisée par le fait que** le nombre de motifs oxyalkylénés varie de 7 à 25.

27. Composition selon l'une quelconque des revendications 1 à 19, **caractérisée par le fait que** le polymère amphiphile d'AMPS est choisi parmi :
- les copolymères réticulés ou non réticulés, neutralisés ou non, comportant de 15 à 60% en poids de motifs AMPS et de 40 à 85% en poids de motifs (C₈-C₁₆)alkyl(mêth)acrylamide ou de motifs (C₈-C₁₆)alkyl(meth)acrylate par rapport au polymère ;
- les terpolymères comportant de 10 à 90% en mole de motifs acrylamide, de 0,1 à 10% en mole de motifs AMPS et de 5 à 80% en mole de motifs n-(C₈₋₁₈)alkylacrylamide, par rapport au polymère.

28. Composition selon l'une quelconque des revendications 1 à 19, **caractérisée par le fait que** le polymère amphiphile d'AMPS est choisi parmi :
- les copolymères non réticulés et réticulés d'AMPS partiellement ou totalement neutralisé et de méthacrylate de n-dodécyle.
- les copolymères non réticulés et réticulés d'AMPS partiellement ou totalement neutralisé et de n-dodécylméthacylamide.

29. Composition selon les revendications 1 à 26 **caractérisée par le fait que** le polymère amphiphile d'AMPS est choisi parmi les copolymères constitués de motifs acide 2-acrylamido-2-méthylpropane-sulfonique (AMPS) de formule (II) suivante: 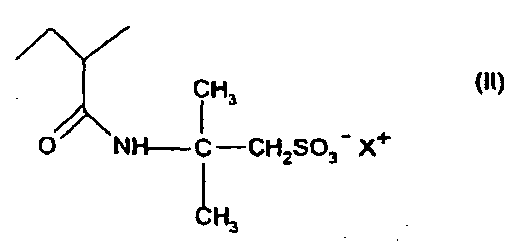 dans laquelle X⁺ est un proton, un cation de métal alcalin, un cation alcallno-terreux ou l'ion ammonium,
et de motifs de formule (III) suivante : 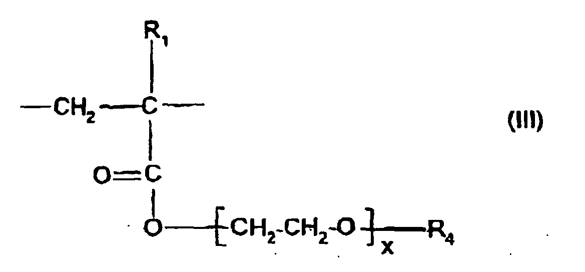 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₈-C₂₂ et plus préférentiellement en C₁₀-C₂₂.

30. Composition selon la revendication 29, **caractérisée par le fait que** x = 25 , R₁ est méthyle et R₄ est n-dodécyle.

31. Composition selon la revendication 1 ou 29, **caractérisée par le fait que** la proportion molaire en % des motifs de formule (I) ou des motifs de formule (III) dans les polymères varie de 50,1 à 99,9%.

32. Composition selon la revendication 1 ou 29, **caractérisée par le fait que** la proportion molaire en % des motifs de formule (I) ou des motifs de formule (III) dans les polymères varie de 0,1 à 50%.

33. Composition selon l'une quelconque des revendications précédentes, **caractérisée par le fait que** les polymères amphiphiles sont présents dans des concentrations allant de 0.01 à 30% en poids.

34. Composition selon l'une quelconque des revendications précédentes, **caractérisée par le fait que** les polymères amphiphiles sont présents dans des concentrations allant 0,1 à 10% en poids par rapport au poids total de la composition.

35. Composition selon l'une quelconque des revendications précédentes, **caractérisée par le fait que** les polymères amphiphiles sont présents dans des concentrations allant de 0,1 à 5% en poids par rapport au poids total de la composition.

36. Composition selon l'une quelconque des revendications précédentes, **caractérisée par le fait que** les polymères amphiphiles sont présents dans des concentrations allant de 0,5 à 2% en poids par rapport au poids total de la composition.

37. Composition selon l'une quelconque des revendications 1 à 36, **caractérisée par le fait que** l'agent oxydant est choisi dans le groupe formé par le peroxyde d'hydrogène, le peroxyde d'urée, les perborates, les persulfates, ou leurs mélanges.

38. Composition selon l'une quelconque des revendications 1 à 37, **caractérisée par le fait que** l'agent oxydant est le peroxyde d'hydrogène.

39. Composition selon la revendication 37 ou 38, **caractérisée par le fait que** la concentration en peroxyde d'hydrogène varie de 0,5 à 40 volumes et de préférence de 2 à 30 volumes.

40. Composition selon la revendication 38, **caractérisée par le fait que** l'eau oxygénée contient un agent stabilisant

41. Composition selon la revendication 40, **caractérisée par le fait que** l'agent stabilisant est choisi parmi les pyrophosphates de métaux alcalins ou alcalino-terreux, les stannates des métaux alcalins ou alcalino-terreux, la phénacétine, ou les sels d'acides et d'oxyquinoleine.

42. Composition selon la revendication 41, **caractérisée par le fait que** ragent stabilisant est un stannate ou un stannate associé à un pyrophosphate.

43. Composition selon l'une quelconque des revendications 40 à 42, **caractérisée par le fait que** la concentration en agent stabilisant varie de 0,0001 à 5% en poids par rapport au poids total de la composition.

44. Composition selon l'une quelconque des revendications 38 à 43, **caractérisée par le fait que** le rapport des concentrations du peroxyde d'hydrogène aux agents stabilisants varie de 0,05 à 1000.

45. Composition selon l'une quelconque des revendications 38 à 44, **caractérisée par le fait que** le rapport des concentrations du ou des polymères amphiphiles définis selon les revendications 1 à 36, agents stabilisants, varie de 0,05 à 1000,

46. Composition selon l'une quelconque des revendications précédentes, **caractérisée par le fait que** le rapport des concentrations du ou des polymères amphiphiles définis selon les revendications 1 à 36, aux agents oxydants, varie de 0,001 à 10,

47. Composition selon l'une quelconque des revendications 37 à 45, **caractérisée par le fait que** la concentration en agents oxydants varie de 0,1 à 25% en poids par rapport au poids total de la composition.

48. Composition selon l'une quelconque des revendications 1 à 47, **caractérisée par le fait qu'**elle est aqueuse et que le pH de l'ensemble de la composition varie de 1 à 13.

49. Composition selon l'une quelconque des revendications 1 à 48, **caractérisée par le fait qu'**elle se présente sous la forme d'un gel transparent de viscosité allant de 50 mPa.s à 10 Pa.s.

50. Composition selon l'une quelconque des revendications 1 à 49, **caractérisée par le fait qu'**elle se présente sous la forme d'un gel transparent de viscosité allant de 75 mPa.s à 0.5 Pa.s.

51. Procédé de teinture d'oxydation des fibres kératiniques humaines et en particulier les cheveux mettant en oeuvre une composition colorante comprenant dans un support approprié pour la teinture des fibres kératiniques au moins un colorant d'oxydation et une composition oxydante telle que définie dans l'une quelconque des revendications 1 à 50.

52. Procédé de teinture selon la revendication 51 selon lequel on mélange, au moment de l'emploi, la composition colorante avec la composition oxydante ; le mélange obtenu est ensuite appliqué sur les fibres kératiniques et on laisse poser pendant 3 à 50 minutes environ, de préférence 5 à 30 minutes environ, après quoi on rince, on lave au

53. Procédé de teinture selon la revendication 51 selon lequel on applique séquentiellement la composition colorante et la composition oxydante, avec ou sans rinçage intermédiaire.

54. Procédé de traitement des fibres kératiniques humaines et en particulier les cheveux, en vue d'obtenir une déformation permanente de ces dernières, en particulier sous la forme de cheveux permanentes, ce procédé comprenant les étapes suivantes : (i) on applique sur la matière kératinique à traiter une composition réductrice, la fibre kératinique étant mise sous tension mécanique avant, pendant, ou après ladite application, (ii) on rince éventuellement la fibre kératinique, (iii) on applique sur la fibre kératinique éventuellement rincée une composition oxydante telle que définie à l'une des revendications 1 à 50, (iv) on rince éventuellement à nouveau la fibre kératinique.

55. Procédé de décoloration des fibres kératiniques humaines et en particulier les cheveux, comprenant les étapes suivantes : i) on applique sur la fibre kératinique une composition oxydante selon l'une quelconque des revendications 1 à 50, ii) on rince la fibre kératinique ainsi traitée.

56. Utilisation comme agent épaississant et/ou gélifiant dans une composition cosmétique et/ou dermatologique comprenant au moins un oxydant, d'un polymère amphiphile tel que défini à l'une quelconque des revendications 1 à 34.

## Claims

1. Cosmetic and/or dermatological composition for treating keratin materials, **characterized in that** it comprises, in a support that is suitable for keratin materials:
(a) at least one amphiphilic polymer comprising at least one ethylenically unsaturated monomer containing a sulfonic group, in free form or partially or totally neutralized form, and also at least one hydrophobic portion chosen from amphiphilic copolymers of AMPS and of at least one ethylenically unsaturated hydrophobic monomer comprising at least one hydrophobic portion containing from 6 to 50 carbon atoms, and chosen from the acrylates or acrylamides of formula (I) below: 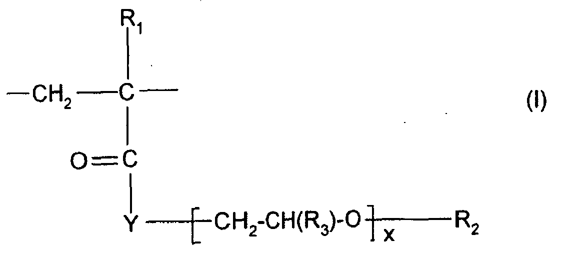 in which R₁ and R₃, which may be identical or different, denote a hydrogen atom or a linear or branched C₁-C₆ alkyl radical; Y denotes O or NH; R₂ denotes a hydrophobic hydrocarbon radical containing at least from 6 to 50 carbon atoms; x denotes a number of moles of alkylene oxide and ranges from 0 to 100, and
(b) at least one oxidizing agent.

2. Composition according to Claim 1, **characterized in that** R₂ denotes a hydrophobic hydrocarbon radical containing from 6 to 22 carbon atoms.

3. Composition according to Claim 2, **characterized in that** R₂ denotes a hydrophobic hydrocarbon radical containing from 6 to 18 carbon atoms.

4. Composition according to Claim 3, **characterized in that** R₂ denotes a hydrophobic hydrocarbon radical containing from 12 to 18 carbon atoms.

5. Composition according to any one of Claims 1 to 4, **characterized in that** the amphiphilic polymers are partially or totally neutralized with a mineral or organic base.

6. Composition according to any one of Claims 1 to 5, **characterized in that** the amphiphilic polymers have a number-average molecular weight ranging from 1000 to 20 000 000 g/mol.

7. Composition according to Claim 6, **characterized in that** the number-average molecular weight ranges from 20 000 to 5 000 000 g/mol.

8. Composition according to Claim 7, **characterized in that** the number-average molecular weight ranges from 100 000 to 1 500 000 g/mol.

9. Composition according to any one of the preceding claims, **characterized in that** an aqueous solution at 1% by weight of said polymers has, at a temperature of 25°C, a viscosity, measured using a Brookfield viscometer with a No. 7 needle, ranging from 20 000 mPa.s to 100 000 mPa.s.

10. Composition according to any one of the preceding claims, **characterized in that** the amphiphilic polymers are prepared by free-radical precipitation polymerization in tert-butanol.

11. Composition according to any one of the preceding claims, **characterized in that** the amphiphilic polymers are crosslinked or non-crosslinked.

12. Composition according to Claim 11, **characterized in that** the amphiphilic polymers are crosslinked.

13. Composition according to Claim 12, **characterized in that** the crosslinking agent(s) is (are) chosen from polyolefinically unsaturated compounds.

14. Composition according to Claim 13, **characterized in that** the crosslinking agent(s) is (are) chosen from methylenebisacrylamide, allyl methacrylate and trimethylolpropane triacrylate (TMPTA).

15. Composition according to any one of Claims 12 to 14, **characterized in that** the degree of crosslinking preferably ranges from 0.01 mol% to 10 mol%.

16. Composition according to any one of Claims 1 to 15, **characterized in that** the amphiphilic polymers are chosen from random AMPS polymers modified by reaction with an n-mono(C₆-C₂₂)alkylamine or a di-n-(C₆-C₂₂)alkylamine.

17. Composition according to any one of Claims 1 to 16, **characterized in that** the amphiphilic AMPS polymers also contain at least one ethylenically unsaturated monomer not comprising a fatty chain.

18. Composition according to Claim 17, **characterized in that** the ethylenically unsaturated monomer not comprising a fatty chain is chosen from (meth)acrylic acids and β-substituted alkyl derivatives thereof, and esters thereof obtained with monoalcohols or mono- or polyalkylene glycols, or alternatively from (meth)acrylamides, vinylpyrrolidone, maleic anhydride, itaconic acid or maleic acid, or mixtures of these compounds.

19. Composition according to any one of Claims 1 to 18, **characterized in that** the hydrophobic radical R₂ is chosen from linear, branched or cyclic C₆-C₁₈ alkyl radicals; C₆-C₁₈ alkylperfluoro radicals; the cholesteryl radical or a cholesterol ester; aromatic polycyclic groups.

20. Composition according to any one of Claims 1 to 19, **characterized in that** the monomer of formula (I) also comprises at least one alkylene oxide unit (x ≥ 1).

21. Composition according to any one of Claims 1 to 20, **characterized in that** the monomer of formula (I) also comprises at least one polyoxyalkylenated chain.

22. Composition according to Claim 21, **characterized in that** the polyoxyalkylenated chain consists of ethylene oxide units and/or of propylene oxide units.

23. Composition according to Claim 22, **characterized in that** the polyoxyalkylenated chain consists solely of ethylene oxide units.

24. Composition according to any one of Claims 1 to 23, **characterized in that** the number of oxyalkylene units ranges from 3 to 100.

25. Composition according to Claim 24, **characterized in that** the number of oxyalkylene units ranges from 3 to 50.

26. Composition according to Claim 25, **characterized in that** the number of oxyalkylene units ranges from 7 to 25.

27. Composition according to any one of Claims 1 to 19, **characterized in that** the amphiphilic AMPS polymer is chosen from:
- crosslinked or non-crosslinked, neutralized or non-neutralized copolymers comprising from 15% to 60% by weight of AMPS units and from 40% to 85% by weight of (C₈-C₁₆)alkyl(meth)acrylamide units or of (C₈-C₁₆)alkyl (meth)acrylate units relative to the polymer;
- terpolymers comprising from 10 mol% to 90 mol% of acrylamide units, from 0.1 mol% to 10 mol% of AMPS units and from 5 mol% to 80 mol% of n-(C₆-C₁₈)alkylacrylamide units relative to the polymer.

28. Composition according to any one of Claims 1 to 19, **characterized in that** the amphiphilic AMPS polymer is chosen from:
- non-crosslinked and crosslinked copolymers of partially or totally neutralized AMPS and of n-dodecyl methacrylate;
- non-crosslinked and crosslinked copolymers of partially or totally neutralized AMPS and of n-dodecylmethacrylamide.

29. Composition according to Claims 1 to 26, **characterized in that** the amphiphilic AMPS polymer is chosen from copolymers consisting of 2-acrylamido-2-methylpropanesulfonic acid (AMPS) units of formula (II) below: 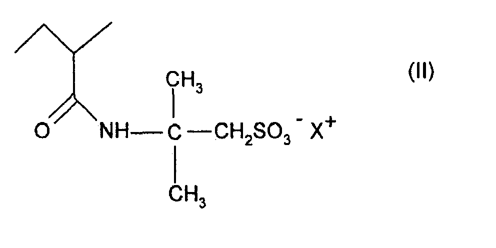 in which X⁺ is a proton, an alkali metal cation, an alkaline-earth metal cation or the ammonium ion,
and of units of formula (III) below: 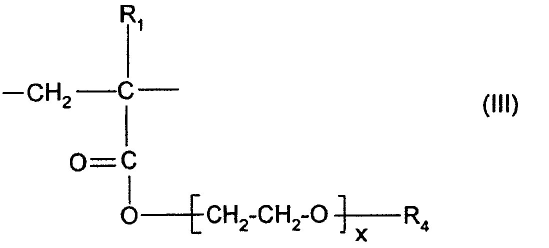 in which x denotes an integer ranging from 3 to 100, preferably from 5 to 80 and more preferably from 7 to 25; R₁ has the same meaning as that given above in formula (I) and R₄ denotes a linear or branched C₆-C₂₂ and more preferably C₁₀-C₂₂ alkyl.

30. Composition according to Claim 29, **characterized in that** x = 25, R₁ is methyl and R₄ is n-dodecyl.

31. Composition according to Claim 1 or 29, **characterized in that** the molar percentage proportion of units of formula (I) or of units of formula (III) in the polymers ranges from 50.1% to 99.9%.

32. Composition according to Claim 1 or 29, **characterized in that** the molar percentage proportion of units of formula (I) or of units of formula (III) in the polymers ranges from 0.1% to 50%.

33. Composition according to any one of the preceding claims, **characterized in that** the amphiphilic polymers are present in concentrations ranging from 0.01% to 30% by weight.

34. Composition according to any one of the preceding claims, **characterized in that** the amphiphilic polymers are present in concentrations ranging from 0.1% to 10% by weight relative to the total weight of the composition.

35. Composition according to any one of the preceding claims, **characterized in that** the amphiphilic polymers are present in concentrations ranging from 0.1% to 5% by weight relative to the total weight of the composition.

36. Composition according to any one of the preceding claims, **characterized in that** the amphiphilic polymers are present in concentrations ranging from 0.5% to 2% by weight relative to the total weight of the composition.

37. Composition according to any one of Claims 1 to 36, **characterized in that** the oxidizing agent is chosen from the group formed by hydrogen peroxide, urea peroxide, perborates and persulfates, or mixtures thereof.

38. Composition according to any one of Claims 1 to 37, **characterized in that** the oxidizing agent is hydrogen peroxide.

39. Composition according to Claim 37 or 38, **characterized in that** the hydrogen peroxide concentration ranges from 0.5 to 40 volumes and preferably from 2 to 30 volumes.

40. Composition according to Claim 38, **characterized in that** the aqueous hydrogen peroxide solution contains a stabilizer.

41. Composition according to Claim 40, **characterized in that** the stabilizer is chosen from alkali metal or alkaline-earth metal pyrophosphates, alkali metal or alkaline-earth metal stannates, phenacetin or salts of acids and of oxyquinoline.

42. Composition according to Claim 41, **characterized in that** the stabilizer is a stannate or a stannate combined with a pyrophosphate.

43. Composition according to any one of Claims 40 to 42, **characterized in that** the stabilizer concentration ranges from 0.0001% to 5% by weight relative to the total weight of the composition.

44. Composition according to any one of Claims 38 to 43, **characterized in that** the ratio of the concentrations of the hydrogen peroxide to the stabilizers ranges from 0.05 to 1000.

45. Composition according to any one of Claims 38 to 44, **characterized in that** the ratio of the concentrations of the amphiphilic polymer(s) defined according to Claims 1 to 36 to the stabilizers ranges from 0.05 to 1000.

46. Composition according to any one of the preceding claims, **characterized in that** the ratio of the concentrations of the amphiphilic polymer(s) defined according to Claims 1 to 36 to the oxidizing agents ranges from 0.001 to 10.

47. Composition according to any one of Claims 37 to 45, **characterized in that** the concentration of oxidizing agents ranges from 0.1% to 25% by weight relative to the total weight of the composition.

48. Composition according to any one of Claims 1 to 47, **characterized in that** it is aqueous and **in that** the pH of the whole composition ranges from 1 to 13.

49. Composition according to any one of Claims 1 to 48, **characterized in that** it is in the form of a transparent gel with a viscosity ranging from 50 mPa.s to 10 Pa.s.

50. Composition according to any one of Claims 1 to 49, **characterized in that** it is in the form of a transparent gel with a viscosity ranging from 75 mPa.s to 0.5 Pa.s.

51. Process for the oxidation dyeing of human keratin fibres, and in particular the hair, using a dye composition comprising, in a support that is suitable for dyeing keratin fibres, at least one oxidation dye and an oxidizing composition as defined in any one of Claims 1 to 50.

52. Dyeing process according to Claim 51, according to which the dye composition is mixed, at the time of use, with the oxidizing composition; the mixture obtained is then applied to the keratin fibres and is left to act for 3 to 50 minutes approximately and preferably 5 to 30 minutes approximately, after which the fibres are rinsed, washed with shampoo, rinsed again and dried.

53. Dyeing process according to Claim 51, according to which the dye composition and the oxidizing composition are applied sequentially, with or without intermediate rinsing.

54. Process for treating human keratin fibres, and in particular the hair, to permanently reshape these fibres, in particular in the form of permanent-waved hair, this process comprising the following steps: (i) a reducing composition is applied to the keratin material to be treated, the keratin fibre being placed under mechanical tension before, during or after said application, (ii) the keratin fibre is optionally rinsed, (iii) an oxidizing composition as defined in one of Claims 1 to 50 is applied to the optionally rinsed keratin fibre, (iv) the keratin fibre is optionally rinsed again.

55. Process for bleaching human keratin fibres, and in particular the hair, comprising the following steps: i) an oxidizing composition according to any one of Claims 1 to 50 is applied to the keratin fibre, ii) the keratin fibre thus treated is rinsed.

56. Use, as a thickener and/or gelling agent in a cosmetic and/or dermatological composition comprising at least one oxidizing agent, of an amphiphilic polymer as defined in any one of Claims 1 to 34.

## Patentansprüche

1. Kosmetische und/oder dermatologische Zusammensetzung, die für die Behandlung von Keratinsubstanzen vorgesehen ist, **dadurch gekennzeichnet, dass** sie in einem für Keratinsubstanzen geeigneten Träger enthält:
(a) mindestens ein amphiphiles Polymer, das mindestens ein Monomer mit ethylenisch ungesättigter Bindung, das eine Sulfonsäuregruppe in freier Form oder ganz oder teilweise neutralisiert aufweist, und ferner mindestens einen hydrophoben Bereich enthält und das ausgewählt ist unter den amphiphilen Copolymeren von AMPS und mindestens einem hydrophoben Monomer mit ethylenisch ungesättigter Bindung, das mindestens einen hydrophoben Bereich mit 6 bis 50 Kohlenstoffatomen enthält, welches unter den Acrylaten oder Acrylamiden der folgenden Formel (I) ausgewählt ist: 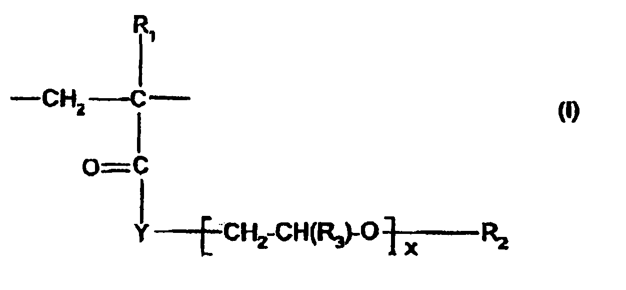 worin die Gruppen R₁ und R₃, die gleich oder verschieden sind, ein Wasserstoffatom oder eine geradkettige oder verzweigte C₁₋₆Alkylgruppe bedeuten; Y O oder NH ist; R₂ eine hydrophobe Kohlenwasserstoffgruppe bedeutet, die mindestens 6 bis 50 Kohlenstoffatome aufweist; und x die Molzahl an Alkylenoxid angibt und im Bereich von 0 bis 100 liegt, und
(b) mindestens ein Oxidationsmittel.

2. Zusammensetzung nach Anspruch 1, **dadurch gekennzeichnet, dass** R₂ eine hydrophobe Kohlenwasserstoffgruppe mit 6 bis 22 Kohlenstoffatomen bedeutet.

3. Zusammensetzung nach Anspruch 2, **dadurch gekennzeichnet, dass** R₂ eine hydrophobe Kohlenwasserstoffgruppe mit 6 bis 18 Kohlenstoffatomen ist.

4. Zusammensetzung nach Anspruch 3, **dadurch gekennzeichnet, dass** R₂ eine hydrophobe Kohlenwasserstoffgruppe mit 12 bis 18 Kohlenstoffatomen bedeutet.

5. Zusammensetzung nach einem der Ansprüche 1 bis 4, **dadurch gekennzeichnet, dass** die amphiphilen Polymere ganz oder teilweise mit einer anorganischen oder organischen Base neutralisiert sind.

6. Zusammensetzung nach einem der Ansprüche 1 bis 5, **dadurch gekennzeichnet, dass** die amphiphilen Polymere eine zahlenmittlere Molmasse von 1000 bis 20 000 000 g/mol aufweisen.

7. Zusammensetzung nach Anspruch 6, **dadurch gekennzeichnet, dass** die zahlenmittlere Molmasse im Bereich von 20 000 bis 5 000 000 g/mol liegt.

8. Zusammensetzung nach Anspruch 7, **dadurch gekennzeichnet, dass** die zahlenmittlere Molmasse im Bereich von 100 000 bis 1 500 000 g/mol liegt.

9. Zusammensetzung nach einem der vorhergehenden Ansprüche, **dadurch gekennzeichnet, dass** eine wässrige Lösung von 1 Gew.-% der Polymere bei einer Temperatur von 25 °C eine mit einem Brookfield-Viskosimeter, Nadel 7, gemessene Viskosität von 30 000 bis 100 000 mPa·s aufweist.

10. Zusammensetzung nach einem der vorhergehenden Ansprüche, **dadurch gekennzeichnet, dass** die amphiphilen Polymere durch radikalische Polymerisation unter Ausfällen in t-Butanol hergestellt werden.

11. Zusammensetzung nach einem der vorhergehenden Ansprüche, **dadurch gekennzeichnet, dass** die amphiphilen Polymere vernetzt oder nicht vernetzt sind.

12. Zusammensetzung nach Anspruch 11, **dadurch gekennzeichnet, dass** die amphiphilen Polymere vernetzt sind.

13. Zusammensetzung nach Anspruch 12, **dadurch gekennzeichnet, dass** das oder die Vernetzungsmittel unter den Verbindungen mit mehreren olefinisch ungesättigten Bindungen ausgewählt sind.

14. Zusammensetzung nach Anspruch 13, **dadurch gekennzeichnet, dass** das oder die Vernetzungsmittel unter Methylen-bis-acrylamid, Allylmethacrylat oder Trimethylolpropantriacrylat (TMPTA) ausgewählt sind.

15. Zusammensetzung nach einem der Ansprüche 12 bis 14, **dadurch gekennzeichnet, dass** der Vernetzungsgrad vorzugsweise im Bereich von 0,01 bis 10 Mol-% liegt.

16. Zusammensetzung nach einem der Ansprüche 1 bis 15, **dadurch gekennzeichnet, dass** die amphiphilen Polymere unter den statistischen AMPS-Polymeren ausgewählt sind, die durch Umsetzung mit einem *n*-Mono(C₆₋₂₂)alkylamin oder Di-*n*-(C₆₋₂₂)alkylamin modifiziert sind.

17. Zusammensetzung nach einem der Ansprüche 1 bis 16, **dadurch gekennzeichnet, dass** die amphiphilen AMPS-Polymere ferner mindestens ein Monomer mit ethylenisch ungesättigter Bindung enthalten, das keine Fettkette aufweist.

18. Zusammensetzung nach Anspruch 17, **dadurch gekennzeichnet, dass** das Monomer mit ethylenisch ungesättigter Bindung, das keine Fettkette aufweist, unter (Meth)acrylsäure und deren in β-Stellung substituierten Alkylderivaten und ihren Estern, die mit Monoalkoholen oder Mono- oder Polyalkylenglycolen gebildet werden, oder unter (Meth)acrylamid, Vinylpyrrolidon, Maleinsäureanhydrid, Itaconsäure oder Maleinsäure oder den Gemischen dieser Verbindungen ausgewählt ist.

19. Zusammensetzung nach einem der Ansprüche 1 bis 18, **dadurch gekennzeichnet, dass** die hydrophobe Gruppe R₂ unter den geradkettigen, verzweigten oder cyclischen C₆₋₁₈-Alkylgruppen; C₆₋₁₈-alkylperfluorierten Gruppen; der Cholesterylgruppe oder einem Cholesterinester; aromatischen polycyclischen Gruppen ausgewählt ist.

20. Zusammensetzung nach einem der Ansprüche 1 bis 19, **dadurch gekennzeichnet, dass** das Monomer der Formel (I) ferner mindestens eine Alkylenoxideinheit (x ≥ 1) aufweist.

21. Zusammensetzung nach einem der Ansprüche 1 bis 20, **dadurch gekennzeichnet, dass** das Monomer der Formel (I) ferner mindestens eine polyalkoxylierte Kette enthält.

22. Zusammensetzung nach Anspruch 21, **dadurch gekennzeichnet, dass** die polyalkoxylierte Kette aus Ethylenoxideinheiten und/oder Propylenoxideinheiten besteht.

23. Zusammensetzung nach Anspruch 22, **dadurch gekennzeichnet, dass** die polyalkoxylierte Kette ausschließlich aus Ethylenoxideinheiten besteht.

24. Zusammensetzung nach einem der Ansprüche 1 bis 23, **dadurch gekennzeichnet, dass** die Anzahl der Oxyalkyleneinheiten im Bereich von 3 bis 100 liegt.

25. Zusammensetzung nach Anspruch 24, **dadurch gekennzeichnet, dass** die Anzahl der Oxyalkyleneinheiten im Bereich von 3 bis 50 liegt.

26. Zusammensetzung nach Anspruch 25, **dadurch gekennzeichnet, dass** die Anzahl der Oxyalkyleneinheiten im Bereich von 7 bis 25 liegt.

27. Zusammensetzung nach einem der Ansprüche 1 bis 19, **dadurch gekennzeichnet, dass** das amphiphile AMPS-Polymer ausgewählt ist unter:
- vernetzten oder nicht vernetzten, neutralisierten oder nicht neutralisierten Copolymeren, die 15 bis 60 Gew.-% AMPS-Einheiten und 40 bis 85 Gew.-% (C₈₋₁₆)Alkyl(meth)acrylamideinheiten oder (C₈₋₁₆)Alkyl(meth)acrylateinheiten, bezogen auf das Polymer, enthalten;
- Terpolymere, die 10 bis 90 Mol-% Acrylamideinheiten, 0,1 bis 10 Mol-% AMPS-Einheiten und 5 bis 80 Mol-% *n*-(C₆₋₁₈)Alkylacrylamideinheiten, bezogen auf das Polymer, enthalten.

28. Zusammensetzung nach einem der Ansprüche 1 bis 19, **dadurch gekennzeichnet, dass** das amphiphile AMPS-Polymer ausgewählt ist unter:
- nicht vernetzten und vernetzten Copolymeren von ganz oder teilweise neutralisierter AMPS und *n*-Dodecylacrylat;
- nicht vernetzten und vernetzten Copolymeren von ganz oder teilweise neutralisierter AMPS und *n*-Dodecylmethacrylamid.

29. Zusammensetzung nach den Ansprüchen 1 bis 26, **dadurch gekennzeichnet, dass** das amphiphile AMPS-Polymer unter den Copolymeren ausgewählt ist, die aus 2-Acrylamido-2-methylpropan-sulfonsäureeinheiten (AMPS) der folgenden Formel (II): 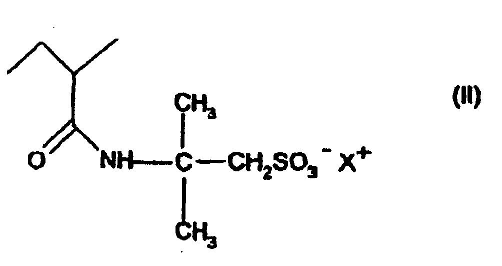 worin X⁺ ein Proton, ein Alkalimetallkation, ein Erdalkalimetallkation oder Ammonium bedeutet,
und Einheiten der folgenden Formel (III) bestehen: 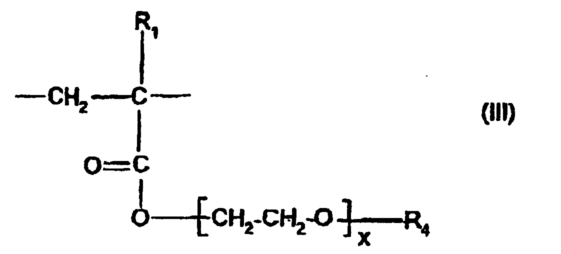 worin x eine ganze Zahl von 3 bis 100, vorzugsweise 5 bis 80 und noch bevorzugter 7 bis 25 bedeutet; R₁ die oben für Formel (I) angegebenen Bedeutungen hat und R₄ eine geradkettige oder verzweigte C₆₋₂₂-Alkylgruppe und vorzugsweise C₁₀₋₂₂-Alkylgruppe ist.

30. Zusammensetzung nach Anspruch 29, **dadurch gekennzeichnet, dass** x = 25, R₁ = Methyl und R₄ = *n*-Dodecyl.

31. Zusammensetzung nach Anspruch 1 oder 29, **dadurch gekennzeichnet, dass** der prozentuale molare Anteil der Einheiten der Formel (I) oder der Einheiten der Formel (III) in den Polymeren im Bereich von 50,1 bis 99,9 % liegt.

32. Zusammensetzung nach Anspruch 1 oder 29, **dadurch gekennzeichnet, dass** der prozentuale molare Anteil der Einheiten der Formel (I) oder der Einheiten der Formel (III) in den Polymeren im Bereich von 0,1 bis 50 % liegt.

33. Zusammensetzung nach einem der vorhergehenden Ansprüche, **dadurch gekennzeichnet, dass** die amphiphilen Polymere in Konzentrationen von 0,01 bis 30 Gew.-% enthalten sind.

34. Zusammensetzung nach einem der vorhergehenden Ansprüche, **dadurch gekennzeichnet, dass** die amphiphilen Polymere in Konzentrationen von 0,1 bis 10 Gew.-%, bezogen auf das Gesamtgewicht der Zusammensetzung, enthalten sind.

35. Zusammensetzung nach einem der vorhergehenden Ansprüche, **dadurch gekennzeichnet, dass** die amphiphilen Polymere in Konzentrationen von 0,1 bis 5 Gew.-%, bezogen auf das Gesamtgewicht der Zusammensetzung, enthalten sind.

36. Zusammensetzung nach einem der vorhergehenden Ansprüche, **dadurch gekennzeichnet, dass** die amphiphilen Polymere in Konzentrationen von 0,5 bis 2 Gew.-%, bezogen auf das Gesamtgewicht der Zusammensetzung, enthalten sind.

37. Zusammensetzung nach einem der Ansprüche 1 bis 36, **dadurch gekennzeichnet, dass** das Oxidationsmittel unter Wasserstoffperoxid, Harnstoffperoxid, Perboraten, Persulfaten oder deren Gemischen ausgewählt ist.

38. Zusammensetzung nach einem der Ansprüche 1 bis 37, **dadurch gekennzeichnet, dass** das Oxidationsmittel das Wasserstoffperoxid ist.

39. Zusammensetzung nach Anspruch 37 oder 38, **dadurch gekennzeichnet, dass** die Wasserstoffperoxidkonzentration im Bereich von 0,5 bis 40 Volumina und vorzugsweise 2 bis 30 Volumina liegt.

40. Zusammensetzung nach Anspruch 38, **dadurch gekennzeichnet, dass** die Wasserstoffperoxidlösung einen Stabilisator enthält.

41. Zusammensetzung nach Anspruch 40, **dadurch gekennzeichnet, dass** der Stabilisator unter den Alkali- oder Erdalkalipyrophosphaten, Alkali- oder Erdalkalistannaten, Phenacetin oder den Salzen von Säuren und Oxychinolin ausgewählt sind.

42. Zusammensetzung nach Anspruch 41, **dadurch gekennzeichnet, dass** der Stabilisator ein Stannat oder ein Stannat in Kombination mit einem Pyrophosphat ist.

43. Zusammensetzung nach einem der Ansprüche 40 bis 42, **dadurch gekennzeichnet, dass** die Konzentration des Stabilisators im Bereich von 0,0001 bis 5 Gew.-%, bezogen auf das Gesamtgewicht der Zusammensetzung, liegt.

44. Zusammensetzung nach einem der Ansprüche 38 bis 43, **dadurch gekennzeichnet, dass** das Konzentrationsverhältnis von Wasserstoffperoxid und Stabilisatoren im Bereich von 0,05 bis 1000 liegt.

45. Zusammensetzung nach einem der Ansprüche 38 bis 44, **dadurch gekennzeichnet, dass** das Konzentrationsverhältnis des oder der amphiphilen Polymere nach einem der Ansprüche 1 bis 36 und der Stabilisatoren im Bereich von 0,05 bis 1000 liegt.

46. Zusammensetzung nach einem der vorhergehenden Ansprüche, **dadurch gekennzeichnet, dass** das Konzentrationsverhältnis des oder der amphiphilen Polymere nach Anspruch 1 bis 36 und der Oxidationsmittel im Bereich von 0,001 bis 10 liegt.

47. Zusammensetzung nach einem der Ansprüche 37 bis 45, **dadurch gekennzeichnet, dass** die Konzentration der Oxidationsmittel im Bereich von 0,1 bis 25 Gew.-%, bezogen auf das Gesamtgewicht der Zusammensetzung, liegt.

48. Zusammensetzung nach einem der Ansprüche 1 bis 47, **dadurch gekennzeichnet, dass** sie wässrig ist und der pH-Wert der gesamten Zusammensetzung im Bereich von 1 bis 13 liegt.

49. Zusammensetzung nach einem der Ansprüche 1 bis 48, **dadurch gekennzeichnet, dass** sie als transparentes Gel mit einer Viskosität von 50 mPa·s bis 10 Pa.s vorliegt.

50. Zusammensetzung nach einem der Ansprüche 1 bis 49, **dadurch gekennzeichnet, dass** sie in Form eines transparenten Gels mit einer Viskosität von 75 mPa·s bis 0,5 Pa·s vorliegt.

51. Verfahren zum oxidativen Färben von menschlichen Keratinfasern und insbesondere Haaren unter Verwendung einer Farbmittelzusammensetzung, die in einem zum Färben von Keratinfasern geeigneten Träger mindestens einen Oxidationsfarbstoff und eine oxidierende Zusammensetzung nach einem der Ansprüche 1 bis 50 enthält.

52. Verfahren zum Färben nach Anspruch 51, nach dem bei der Anwendung die Farbmittelzusammensetzung mit der oxidierenden Zusammensetzung vermischt wird; das erhaltene Gemisch dann auf die Keratinfasern aufgetragen und etwa 3 bis 50 min und vorzugsweise etwa 5 bis 30 min einwirken gelassen wird, worauf gespült, mit Haarwaschmittel gewaschen, nochmals gespült und getrocknet wird.

53. Verfahren zum Färben nach Anspruch 51, bei dem die Farbmittelzusammensetzung und die oxidierende Zusammensetzung nacheinander aufgebracht werden, wobei zwischendurch gespült oder nicht gespült wird.

54. Verfahren zur Behandlung von menschlichen Keratinfasern und insbesondere Haaren, um diese dauerhaft zu verformen, insbesondere in Form von dauergewellten Haaren, wobei das Verfahren die folgenden Schritte umfasst: (i) auf die zu behandelnde Keratinsubstanz wird eine reduzierende Zusammensetzung aufgetragen, wobei die Keratinfaser vor, während oder nach dieser Anwendung mechanisch unter Spannung gesetzt wird, (ii) die Keratinfaser wird gegebenenfalls gespült, (iii) auf die gegebenenfalls gespülte Keratinfaser wird eine oxidierende Zusammensetzung nach einem der Ansprüche 1 bis 50 aufgetragen, (iv) die Keratinfaser wird gegebenenfalls nochmals gespült.

55. Verfahren zum Entfärben von menschlichen Keratinfasern und insbesondere Haaren, das die folgenden Schritte umfasst: i) auf die Keratinfaser wird eine oxidierende Zusammensetzung nach einem der Ansprüche 1 bis 50 aufgetragen, ii) die so behandelte Keratinfaser wird gespült.

56. Verwendung eines amphiphilen Polymers, wie es in einem der Ansprüche 1 bis 34 definiert ist, als Verdickungsmittel und/oder Gelbildner in einer kosmetischen und/ oder dermatologischen Zusammensetzung, die mindestens ein Oxidationsmittel enthält.
